# EUROPÄISCHE PATENTSCHRIFT

(11) **EP 2 526 114 B1**
(45) Veröffentlichungstag und Bekanntmachung des Hinweises auf die Patenterteilung: **18.06.2014**
(21) Anmeldenummer: 11700457.2
(22) Anmeldetag: 21.01.2011
(51) Int. Cl.: C07K 1/22, C07K 16/00, A61K 39/395

(54) **CHROMATOGRAPHISCHES VERFAHREN ZUR AUFREINIGUNG VON FC ENTHALTENDEN PROTEINEN**
CHROMATOGRAPHIC METHOD FOR PURIFYING FC-CONTAINING PROTEINS
PROCÉDÉ CHROMATOGRAPHIQUE POUR PURIFIER DES PROTÉINES CONTENANT FC

(30) Priorität: 05.08.2010 EP 10171975; 22.01.2010 EP 10151416
(43) Veröffentlichungstag der Anmeldung: 28.11.2012
(73) Patentinhaber: Boehringer Ingelheim International GmbH, 55216 Ingelheim am Rhein (DE)
(72) Erfinder: ECKERMANN, Christian, 55216 Ingelheim am Rhein (DE); AMBROSIUS, Dorothee, 55216 Ingelheim am Rhein (DE); NOTHELFER, Franz, 55216 Ingelheim am Rhein (DE); RATHJEN, Thomas, 55216 Ingelheim am Rhein (DE)
(74) Vertreter: Simon, Elke Anna Maria
(86) Internationale Anmeldenummer: PCT/EP2011/050817
(87) Internationale Veröffentlichungsnummer: WO 2011/089212

(56) Entgegenhaltungen:
- WO-A2-2007/109163
- WO-A2-2008/031020
- US-A1- 2005 176 109
- ARAKAWA T ET AL: "Elution of antibodies from a Protein-A column by aqueous arginine solutions", PROTEIN EXPRESSION AND PURIFICATION, ACADEMIC PRESS, SAN DIEGO, CA LNKD- DOI:10.1016/J.PEP.2004.04.009, Bd. 36, Nr. 2, 1. August 2004 (2004-08-01) , Seiten 244-248, XP004520288, ISSN: 1046-5928

## Beschreibung

Die Erfindung betrifft chromatographische Verfahren zur Reinigung von Proteinen und Mittel für solche Verfahren.

Biomoleküle wie Proteine, Polynukleotide, Polysaccharide und dergleichen gewinnen als Medikamente, als Diagnostika, als Zusatzstoffe in Nahrungsmitteln, Waschmitteln und dergleichen, als Forschungsreagenzien und für viele weitere Anwendungen zunehmend an kommerzieller Bedeutung. Der Bedarf an solchen Biomolekülen lässt sich - z.B. im Falle von Proteinen - in aller Regel nicht mehr durch Isolierung der Moleküle aus natürlichen Quellen befriedigen sondern erfordert den Einsatz biotechnologischer Produktionsmethoden.

Die biotechnologische Herstellung von Proteinen beginnt typischerweise mit der Isolierung der DNA, die für das gewünschte Protein codiert, und deren Klonieren in einen geeigneten Expressionsvektor. Nach Transfektion des Expressionsvektors in geeignete prokaryontische oder eukaryontische Expressionszellen und anschließender Selektion transfizierter Zellen werden letztere in Fermentern kultiviert und das gewünschte Protein zur Expression gebracht. Anschließend erfolgt die Ernte der Zellen bzw. des Kulturüberstandes und die Aufarbeitung und Reinigung des darin enthaltenen Proteins.

Im Falle eukaryontischer Expressionssysteme, also bei Verwendung von Säugetierzellkulturen wie etwa CHO oder NS0-Zellen konnte in den zurückliegenden 15 Jahren eine Steigerung der beim Expressionsschritt erreichbaren Konzentration des gewünschten Proteins in den Zellkulturen bzw. Zellkulturüberständen um den Faktor 100 erzielt werden. Im gleichen Zeitraum stieg die Bindekapazität von Chromatographiematerialien, die bei der anschließenden Aufreinigung der Proteine eingesetzt werden, gerade einmal um den Faktor 3. Aus diesem Grund besteht ein dringender Bedarf an verbesserten, optimierten Aufreinigungsverfahren für Biomoleküle, insbesondere Proteine, die in großem, industriellem Maßstab durchgeführt werden können.

Im Falle von Biopharmazeutika, wie etwa als Medikamente eingesetzten Proteinen, z.B. therapeutischen Antikörpern, ist neben der Produktausbeute auch die Abtrennung von Verunreinigungen von überragender Bedeutung. Hierbei können prozess- und produktabhängige Verunreinigungen unterschieden werden. Die prozessabhängigen Verunreinigungen beinhalten Komponenten der Wirtszellen wie Proteine (Wirtszellproteine, "Host cell proteins", HCP) und Nukleinsäuren und stammen aus der Zellkultur (wie Medienbestandteile) oder aus der Aufarbeitung (wie etwa Salze oder abgelöste Chromatographie-Liganden). Produktabhängige Verunreinigungen sind molekulare Varianten des Produkts mit abweichenden Eigenschaften. Hierzu zählen verkürzte Formen wie Vorläufer und hydrolytische Abbauprodukte, aber auch modifizierte Formen, entstanden beispielsweise durch Desaminierungen, falsche Glykosylierungen oder falsch verknüpfte Disulfidbrücken. Ebenso zu den produktabhängigen Varianten zählen Polymere und Aggregate. Weitere Verunreinigungen sind Kontaminanten. Damit werden alle weiteren Materialien chemischer, biochemischer oder mikrobiologischer Natur bezeichnet, die nicht direkt zum Herstellungsprozess gehören. Kontaminanten sind z.B. Viren, die unerwünschterweise in Zellkulturen auftreten können.

Verunreinigungen führen im Falle von Biopharmazeutika zu Sicherheitsbedenken. Diese werden verstärkt, wenn - wie sehr häufig bei Biopharmazeutika - die Verabreichung der therapeutischen Proteine über Injektion oder Infusion direkt in die Blutbahn erfolgt. So können Wirtszellkomponenten zu allergischen Reaktionen oder immunopathologischen Effekten führen. Daneben können Verunreinigungen auch zu einer unerwünschten Immunogenität des verabreichten Proteins führen, also eine unerwünschte Immunreaktion des Patienten auf das Therapeutikum auslösen, bis hin zu einem lebensbedrohenden anaphylaktischen Schock. Daher besteht ein Bedarf an geeigneten Reinigungsprozessen, mit denen alle unerwünschten Substanzen auf ein unbedenkliches Maß abgereichert werden können. Andererseits können auch im Falle von Biopharmazeutika wirtschaftliche Aspekte nicht ignoriert werden. So sollen die eingesetzten Produktions- und Reinigungsverfahren die Rentabilität des damit erzeugten biopharmazeutischen Produkts nicht gefährden. Zudem spielt auch die Zeit, innerhalb der ein neuer Aufreinigungsprozess etabliert werden kann, eine erhebliche Rolle: Neben dem Einfluss auf die Kosten muss die Prozessentwicklung mit der vorklinischen und klinischen Entwicklung des Medikaments abgestimmt sein. So können z.B. bestimmte präklinische und sämtliche klinischen Studien erst beginnen, wenn hinreichende Mengen des Biopharmazeutikums in hinreichender Reinheit zur Verfügung stehen.

Als Ausgangspunkt für die Entwicklung eines auch in großem Maßstab durchführbaren Aufreinigungsprozesses für einen Antikörper kann z.B. folgender Standard-prozess, bestehend aus vier Grundschritten, dienen: Im ersten Schritt wird das Zielprotein isoliert, aufkonzentriert und stabilisiert ("capturing"). Im zweiten Schritt werden Viren entfernt, im dritten erfolgt eine Reinigung, bei der die meisten Verunreinigungen wie Nukleinsäuren, andere Proteine und Endotoxine abgereichert werden. Im letzten Schritt werden die ggf. noch verbliebenen Spuren von Kontaminanten beseitigt ("polishing").

Neben Filtrations- und Fällungsschritten kommt dabei (säulen-)chromatographischen Verfahren eine ganz zentrale Bedeutung zu. So beinhaltet etwa das "capturing" häufig einen affinitätschromatographischen Reinigungsschritt. Dementsprechend sind heutzutage zahlreiche säulenchromatographische Verfahren und hierbei verwendbare Chromatographiematerialen bekannt.

Affinitätschromatographie-Matrices, nachfolgend auch als Affinitätsmatrices bezeichnet, werden bei der industriellen Reinigung von verschiedenen Substanzen als stationäre Phase eingesetzt. Über immobilisierte Liganden lassen sich Substanzen spezifisch anreichern und reinigen, die eine gewisse Affinität zu dem jeweils verwendeten Liganden besitzen. Für die industrielle Aufreinigung von Antikörpern (Immunglobulinen), insbesondere die Reinigung monoklonaler Antikörper, hat sich die Verwendung von immobilisiertem Protein A als initialen Reinigungsschritt bewährt. Protein A ist ein Protein mit etwa 41 kDA aus *Staphylococcus aureus,* das mit hoher Affinität (10⁻⁸ M - 10⁻¹² M an humanem IgG) an die CH₂/CH₃-Domäne der Fc-region von Immunglobulinen bindet. Bei der Protein-A-Chromatographie binden Immunglobuline oder Fusionsproteine, die ein Protein-A-bindende Fc-Region aufweisen, aus der mobilen Phase spezifisch an den Protein A-Liganden, der kovalent an einen Träger (z.B. Sepharose) gekoppelt ist. Protein A aus *Staphylococcus aureus* (wildtyp Protein A) sowie gentechnisch verändertes, rekombinantes Protein A (rec. Protein A) interagiert über nicht-kovalente Wechselwirkungen mit der konstanten Region (Fc-Fragment) der Antikörper. Diese spezifische Interaktion kann ausgenutzt werden um Verunreinigungen effizient von dem Antikörper abzutrennen. Durch eine Veränderung des pH-Werts kann die Interaktion zwischen Antikörper und Protein A-Liganden gezielt aufgelöst und die Antikörper von der stationären Phase freigesetzt bzw. eluiert werden.

Die Effektivität der Affinitätschromatographie kann gesteigert werden, wenn die stationäre Phase nach Beladung gewaschen wird. Waschen meint hier die Anwendung einer mobilen Phase, die Verunreinigungen von der stationären Phase eluiert, nicht jedoch das Zielprodukt. Im Falle der Affinitätschromatographie von Antikörpern über Protein A-Matrices sind Waschpuffer verwendet worden, die Arginin, Isopropanol, NaCl, oder ein Detergenz enthalten (WO2008031020, W02007109163, W02007081906, W02003066662, Millipore Tech Brief TB1026EN00), nicht jedoch die Kombination dieser Komponenten in einem einzigen Waschpuffer. Eine Kombination von zwei dieser Komponenten, einem Salz und einem Detergens, d.h. ein Polymer wie Z.B. Polyethylene Glykol, Polypropylene Glykol und Copolymere bestehend aus diesen, wurde in dem US Patent 6,870,034 B2 beschrieben.

### Zusammenfassung der Erfindung

Überraschend wurde gefunden, dass eine bestimmte Kombination von Komponenten in einem einzigen Waschpuffer bei der Protein A-Chromatographie zu einer höheren Reinheit eines zu reinigenden Zielproteins führt, als die separate Anwendung der gleichen Komponenten nacheinander. Der erfindungsgemäße Waschpuffer ist darüber hinaus bei der Antikörperreinigung standardmäßig einsetzbar und erlaubt durch den Wegfall von Optimierungsschritten die Verkürzung der Verfahrensentwicklung.

Die Erfindung betrifft ein Verfahren zur Abreicherung von Verunreinigungen aus einer Zusammensetzung, die ein Protein enthält, das die Fc-Domäne eines Immunglobulins aufweist (Zielprotein), durch Protein-A-Chromatographie, mit den Schritten:
a. Anwendung einer mobilen Phase, die das Zielprotein enthält, auf eine stationäre Phase, die Protein A enthält, unter Bedingungen, unter denen das Zielprotein an die stationäre Phase bindet;
b. Anwendung eines Waschpuffers mit einem pH-Wert zwischen 5 und 8 als mobiler Phase, als Zusätze enthaltend
   i. Arginin in einer Konzentration von 0,1 - 1 mol/l,
   ii. Natriumchlorid in einer Konzentration von 0,2 bis 2 mol/l,
   iii. einen Alkohol, ausgewählt aus der Gruppe bestehend aus Isopropanol, n-Propanol, und Ethanol, in einer Konzentration von 5 - 30 % (w/v) und
   iv. Polyvinylpyrrolidon und/oder ein Detergens in einer Konzentration von 0.05 - 2 % (w/v);
c. Anwendung eines Elutionspuffers als mobiler Phase unter Bedingungen, unter denen das Zielprotein von der stationären Phase eluiert wird.

In einem weiteren Aspekt hat der Waschpuffer einen pH-Wert von 6 bis 8.

Bevorzugt beträgt die Argininkonzentration im Waschpuffer 0,4 - 0,6 mol/l, insbesondere 0,5 mol/l. Die Natriumchloridkonzentration im Waschpuffer beträgt bevorzugt 0,9 - 1,1 mol/l, insbesondere 1 mol/l. Als Alkohol im Waschpuffer kommt bevorzugt Isopropanol in einer Konzentration von 10 - 20 % (v/v) zur Anwendung, insbesondere in einer Konzentration von 15 % (v/v).

Polyvinylpyrrolidon (PVP) wird bevorzugt in einer Konzentration von 0,1 - 2 % (w/v) verwendet, insbesondere 0,25 % (w/v). Zusätzlich oder alternativ können Polyoxyethylen-sorbitan-monolaurate (Polysorbat 20, Polysorbat 80) in einer Konzentration von 0,05 - 2 % (w/v) verwendet werden.

In einem weiteren Aspekt betrifft die vorliegende Erfindung einen Waschpuffer für die Affinitätschromatographie mit einem pH-Wert von pH 5 bis pH 8, als enthaltend
i. Arginin in einer Konzentration von 0,1 - 1 mol/l,
ii. Natriumchlorid in einer Konzentration von 0,2 bis 2 mol/l,
iii. einen Alkohol, ausgewählt aus der Gruppe bestehend aus Isopropanol, n-Propanol, und Ethanol, in einer Konzentration von 5 - 30 % (v/v), und
iv. Polyvinylpyrrolidon oder ein Detergens in einer Konzentration von 0.05 - 2 % (w/v); .

### Kurze Beschreibung der Abbildungen

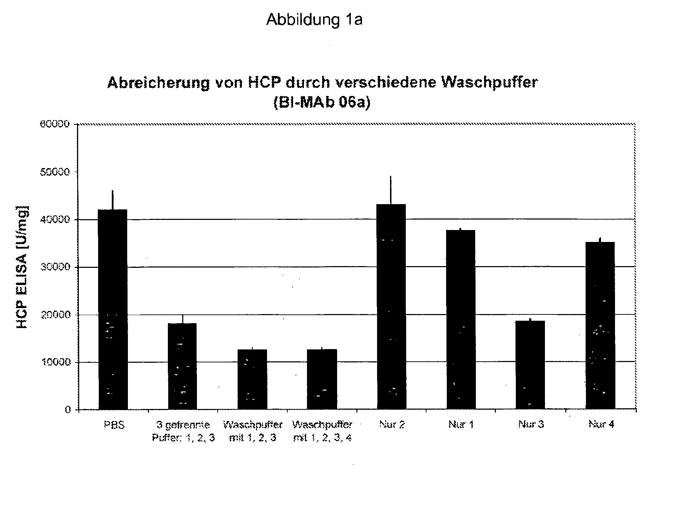

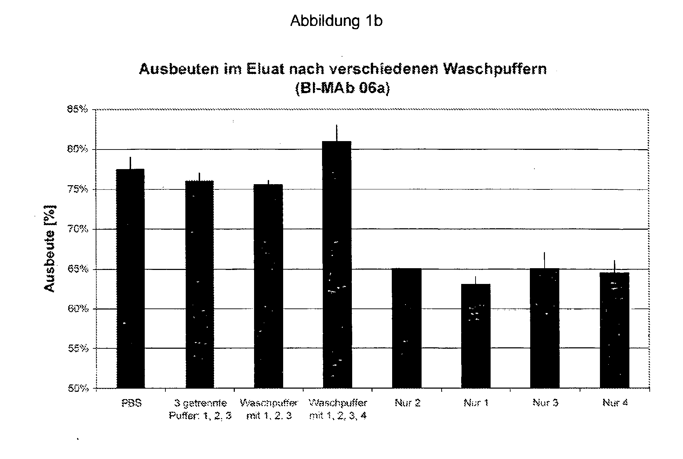

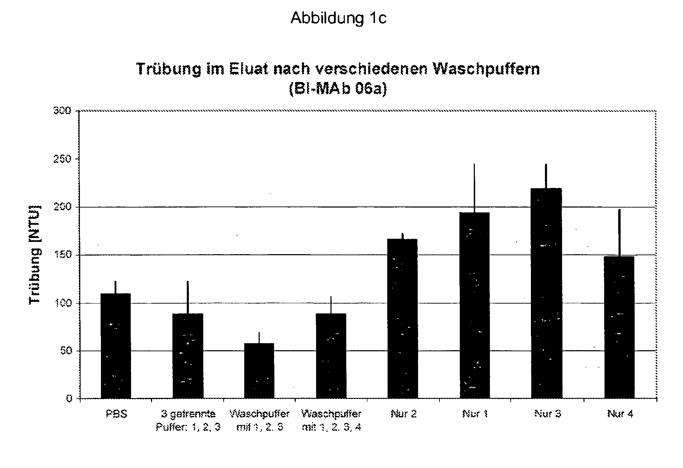

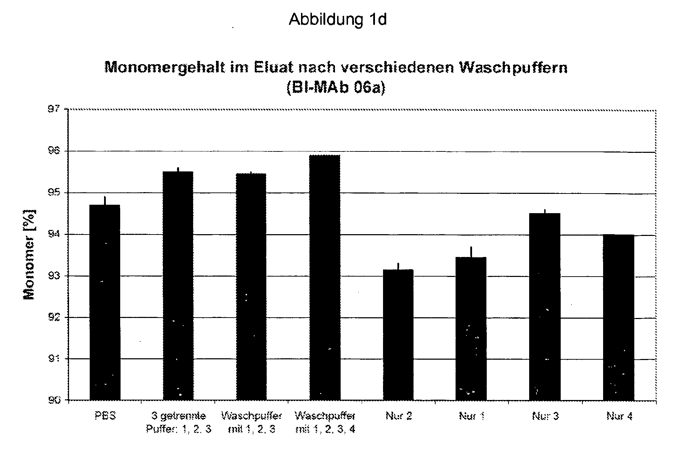

- **Fig. 1**: zeigt die Ausbeuten (1a), Trübungen (1b), Monomergehälter (1c) und Menge an HCP (1d) im Eluat der Affinitätschromatographie nach Waschung mit Waschpuffern unterschiedlicher Kombination und Zusammensetzung am Beispiel des Antikörpers BI-MAb 06a. Die Zahlen auf der Abszisse beziehen sich auf die im Beispiel genannten Zusätze zum Waschpuffer.

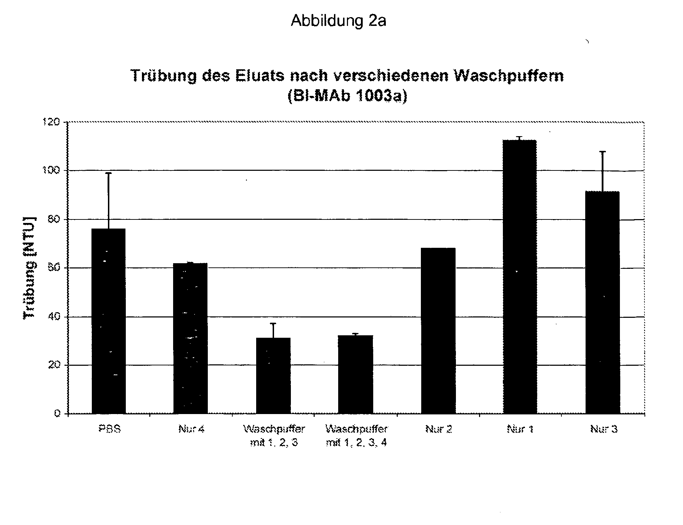

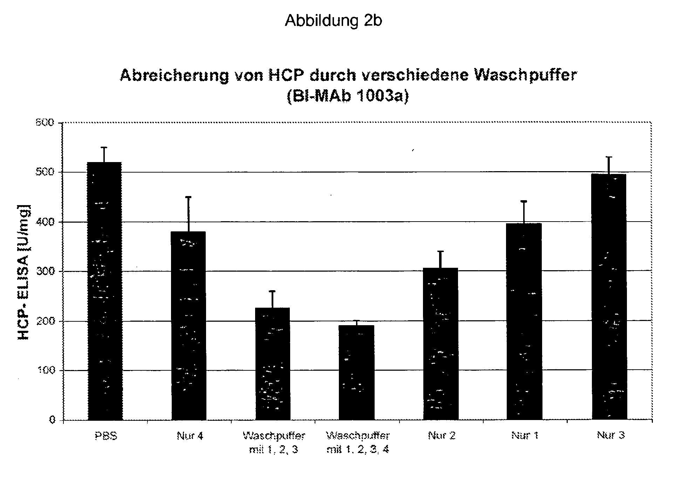

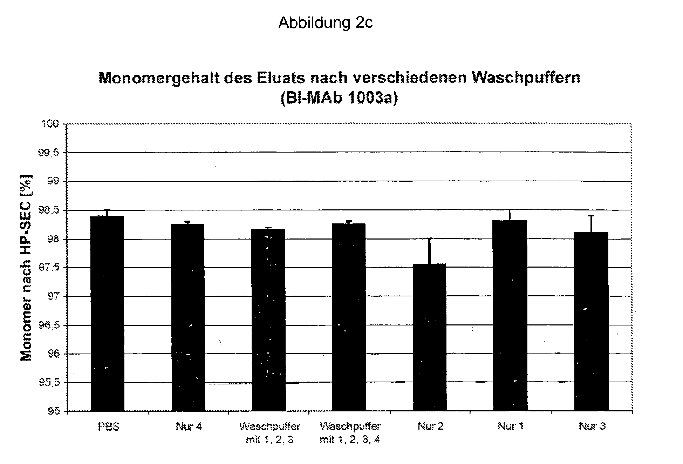

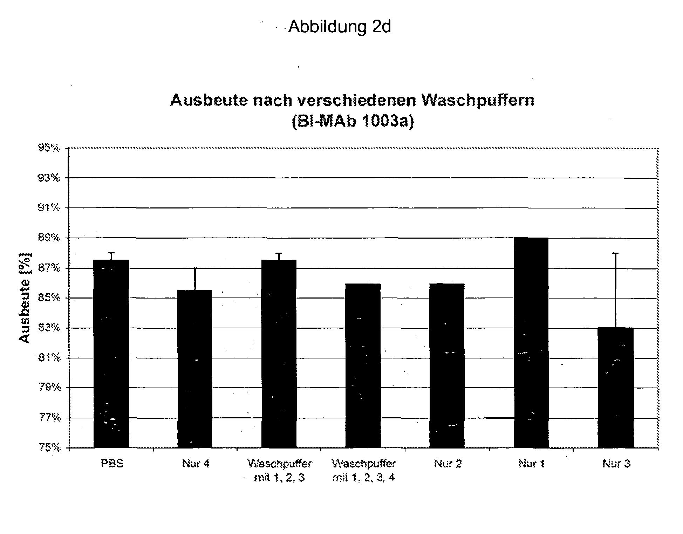

- **Fig. 2**: zeigt die Ausbeuten (2a), Trübungen (2b), Monomergehälter (2c) und Menge an HCP (2d) im Eluat der Affinitätschromatographie nach Waschung mit Waschpuffern unterschiedlicher Kombination und Zusammensetzung am Beispiel des Antikörpers BI-MAb 1003a. Die Zahlen auf der Abszisse beziehen sich auf die im Beispiel genannten Zusätze zum Waschpuffer.

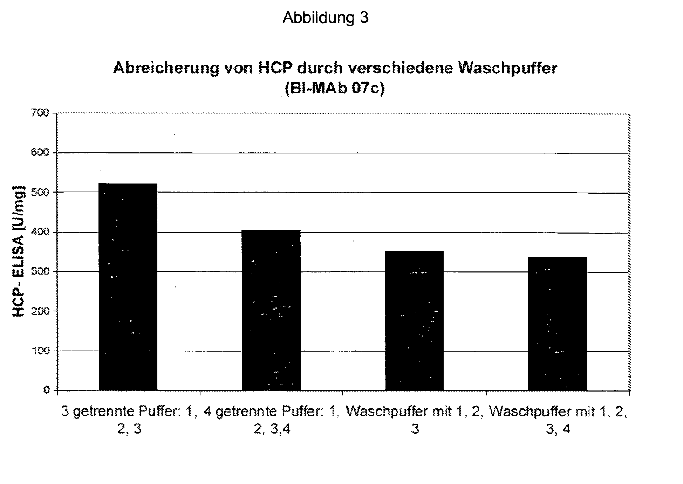

- **Fig. 3**: vergleicht die Menge an HCP im Eluat der Affinitätschromatographie nach Waschung mit Waschpuffern unterschiedlicher Kombination und Zusammesetzung am Beispiel BI-MAb 07c. Die Zahlen auf der Abszisse beziehen sich auf die im Beispiel genannten Zusätze zum Waschpuffer.

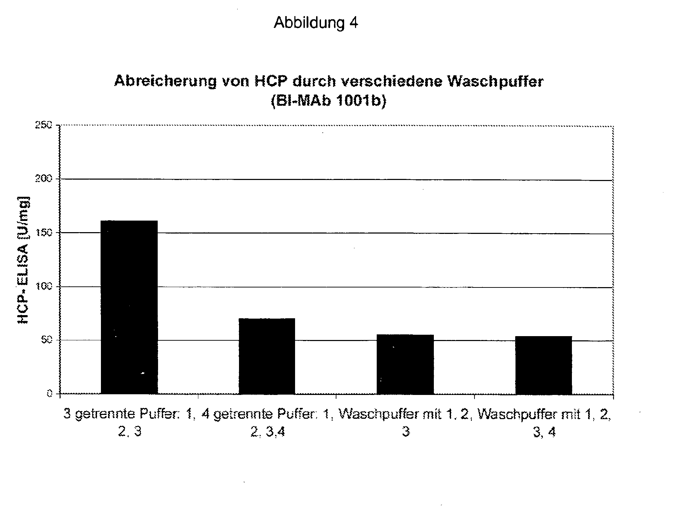

- **Fig. 4**: vergleicht die Menge an HCP im Eluat der Affinitätschromatographie nach Waschung mit Waschpuffern unterschiedlicher Kombination und Zusammesetzung am Beispiel BI-MAb 1001 b. Die Zahlen auf der Abszisse beziehen sich auf die im Beispiel genannten Zusätze zum Waschpuffer.

### Genaue Beschreibung der Erfindung

Die vorliegende Erfindung betrifft Verfahren zur Abreicherung von Verunreinigungen, insbesondere Wirtszellprotein (Host Cell Proteins, HCP) und DNS aus Proteinzusammensetzungen, wie sie aus Zellkulturen erhalten werden, in denen Proteine rekombinant oder endogen exprimiert werden. Insbesondere betrifft die Erfindung Verfahren zur Reinigung bzw. Anreicherung eines Proteins (Zielprotein), die sich über einen Liganden reversibel an einer stationären Phase immobilisieren lassen und so der Affinitätschromatographie zugänglich sind.

Das Zielprotein kann insbesondere ein Immunglobulin oder ein Protein sein, das die Fc-Domäne eines Immunglobulins enthält und an Protein A binden kann. In einer bevorzugten Ausführungsform handelt es sich dabei um Immunglobuline, die aus zwei schweren und zwei leichten Immunglobulinketten bestehen. Antikörper bestehen aus zwei identischen schweren Ketten (heavy chains, H) sowie zwei identischen leichten Ketten (light chains, L), die durch kovalente Disulfidbrücken zu einer Ypsilon-förmigen Struktur miteinander verbunden sind. Die leichten Ketten bestehen aus jeweils einer variablen und einer konstanten Domäne, die als VL und CL bezeichnet werden. Die schweren Ketten andererseits haben jeweils eine variable und drei bis vier konstante Domänen abhängig vom Immunglobulin. Bezeichnet werden diese analog als VH und CH1, CH2, CH3. Die variablen Domänen einer leichten und einer schweren Kette bilden die Antigenbindungsstelle. Die Domäne CH2 enthält eine Kohlenhydratkette, die eine Bindungsstelle für das Komplementsystem bildet. Die CH3 Domäne enthält die Fc-Rezeptor-Bindungsstelle.

Protein A bindet an die Fc-Domäne von Immunglobulinen durch Interaktionen mit der schweren Kette. Die Bindungsaffinität ist am höchsten an humanem IgG1, IgG2 und IgG2a so wie IgG2b von Mäusen. Mit moderater Affinität bindet es an humanem IgM, IgA, IgE und an IgG3 und IgG1 von Mäusen. Es reagiert jedoch weder mit humanem IgG3, IgD noch mit den folgenden Immunglobulinen von Mäusen: IgM, IgA und IgE.

Zielproteine, auf die das erfindungsgemässe Verfahren angewendet werden kann, sind alle Proteine die eine Fc-Domäne aufweisen, beispielsweise Immunglobuline. Immunglobuline können polyklonale oder monoklonale Antikörper sein, die in Hybridomzellen oder rekombinanten Wirtszellen exprimiert werden. Solche Antikörper können ursprünglich durch Immunisierung von Tieren, insbesondere Säugetieren, erzeugt worden sein, einschließlich transgener Tiere, z.B. Mäusen, die die Immunglobuline des Menschen exprimeren. Geeignete Zielproteine sind aber auch Fusionsproteine, bei denen ein beliebiges Protein und die Fc-Domäne eines Immunglobulins fusioniert wurden.

Protein A-Matrices im Sinne der Erfindung sind Affinitätschromatographie-Matrices die immobilisiertes Protein A als Liganden enthalten. Dies umfasst Affinitätsmatrices die wildtyp Protein A, beispielsweise aus *Staphylococcus aureus* als Liganden enthalten. Eine Beschreibung von Protein A befindet sich u.a. bei Lofdahl, S. et al., 1983 (Lindmark, R., Thoren-Tolling, K., Sjoquist J (1983); Binding of immunoglobulins to protein A and immunoglobulin levels in mammalian sera: J Immunol Methods 1983 Aug 12;62(1):1-13.) und Lindmark et. al., 1983 (Lindmark, R., Thoren-Tolling, K., Sjoquist J (1983); Binding of immunoglobulins to protein A and immunoglobulin levels in mammalian sera: J Immunol Methods 1983 Aug 12;62(1):1-13.). Darüber hinaus bezieht sich die Erfindung auch auf Matrices mit rekombinant hergestelltem Protein A als Liganden. Rekombinantes Protein A ist beispielhaft von Duggleby C.J. und Jones, S.A., 1983 (Duggleby, C.J. und Jones, S.A. (1983), Cloning and expression of Staphylococcus aureus protein A gene in Escherichia coli. Nucl.Acid.Res. 1983 May 25; 11 (10):3065-76.) oder Li, R. et al., 1998 (Li, R. Dowd, V., Stewart, D.J., Burton, S.J. Lowe, C.R., Design, sythesis and application of a protein A mimetic. Nat.Biotechnol. 1998 Feb; 16(2):190-5.) beschrieben und dem Fachmann bekannt.

Das Protein A kann hierbei an verschiedene Trägermaterialien wie zum Beispiel an Agarosen, Polysaccharide, Dextrane, Silica-Gele, Glas-beads gekoppelt sein. Eine nicht abschließende Aufstellung geeigneter Trägermaterialien findet sich in Harlow, E. und Lane, D. 1999. Ein häufig verwendetes Trägermaterial bilden auf Agarose basierende Materialien, wie z.B. die dem Fachmann bekannten "Sepharosen" der Firma Amersham Pharmacia Biotech, Uppsala, Schweden. Spezielle Beispiele für Protein A-Sepharosen finden sich im Manual dieser Firma zu dem Thema "Affinity Chromatography" aus dem Jahr 2001. Darüber hinaus sind dem Fachmann weitere Protein A-Chromatographie-Matrices bekannt, wie z.B. MabSelect (Firma Amersham Pharmacia Biotech, Uppsala, Schweden), STREAMLINE™ rProtein A, (Firma Amersham Pharmacia Biotech, Uppsala, Schweden), Poros A (Millipore, Durham, England). Das erfindungsgemäße Verfahren schließt eine Behandlung der entsprechenden Matrices mit ein, wobei die Aufzählung der Matrices beispielhaft und nicht abschließend zu verstehen ist.

Die Kopplung des Liganden erfolgt in der Regel über freie Amino-, Carboxyl- oder Schwefel-Gruppen mittels Cyanogen-Bromid-Aktivierung, NHS-Aktivierung oder Thiol-Kopplung an die Trägermatrix. Siehe hierzu beispielhaft Manual "Affinity Chromatography", Amersham Pharmacia Biotech, Uppsala, Schweden, 2001. In einer besonders bevorzugten Ausführungsform wird Polyvinylpyrrolidon (PVP, auch Polyvidon oder Povidon, CAS: 9003-39-8) in den erfindungsgemäßen Waschpuffern verwendet. Polyvinylpyrrolidon ist ein wasserlösliches Polymer, welches aus N-vinylpyrrolidon-Monomereinheiten besteht. Es kann allerdings auch in anderen polaren Solventen gelöst werden. PVP ist ein hygroskopisches, amorphes Pulver mit weißer bis hellgelber Farbe. Der handelsüblichen Polymere haben eine Molmassen im Bereich von ca. 2500 bis 2.500.000 Dalton. In den Waschpuffern der vorliegenden Erfindung können zusätzlich oder alternativ zu PVP auch Detergenzien verwendet werden. Detergenzien sind oberflächenaktive und amphiphile Moleküle, die Mizellen, also Aggregate von Detergenz-Molekülen, bilden können bei denen der hydrophile Kopf nach außen zum wässrigen Lösungsmittel hin zeigt.

Detergenzien im Sinne der Erfindung sind sowohl nicht-ionische als auch ionische Substanzen. Bevorzugt werden jedoch nicht-ionischen Detergenzien wie z.B. Polyglykolether (Typ NP-40, Tergitol NP40, CAS: 127087-87-0), ein PEG-Alkylether Polyoxyethylen(23)laurylether (CAS: 9002-92-0), PEG-Sorbitanfettsäureester wie Polyoxyethylen(20)sorbitan-monolaurat (Polysorbat 20, CAS: 9005-64-5) oder Polyoxyethylen (20)sorbitan-monooleat (Polysorbat 80, CAS: 9005-65-6), Alkylphenyl-PEG-Ether wie t-Octylphenoxypolyethoxyethanol (Triton-X-100, CAS: 9002-93-1), oder PEO-PPO-Block-Co-Polymere (Poloxamer-Derivate), wie Polyoxyethylen-polyoxypropylen-block-copolymer (Pluronic F 68, Lutrol F 68, Poloxamer 188, CAS: 9003-11-6 oder Poloxamer 407, Pluronic F 127, Lutrol F 127, CAS: 9003-11-6).

Die pH-Werte der Wasch- und Elutionspuffer sind vom Gesamt-System abhängig und werden daherin der Regel für jedes System individuell bestimmt und optimiert.

In einem Aspekt hat der Waschpuffer einen pH-Wert von 5 bis 8. Dem Fachmann ist geläufig, dass der pH-Wert, bei dem ein Protein von einer Säule eluiert, von vielen Faktoren abhängig ist. Unter anderem können dies sein: die Puffer-Systeme während der Bindung, Waschung und Elution, die Anwesenheit von Verunreinigungen, die Geometrie der Matrix-Partikel, die Art der Kopplung des Affinitätsliganden an die Chromatographie-Matrix. Insbesondere haben die spezifischen Eigenschaften des Proteins entscheidenden Einfluss.

In einigen Fällen kann es zu Kombinationen kommen, bei denen es auch oberhalb eines pH-Wertes von pH 4 zu einer teilweisen oder vollständigen Elution des Proteins von dem Affinitätsliganden kommen kann. In einem solchen Fall, und wenn ein Verlust an Protein nicht akzeptabel ist, muss der Waschpuffer einen höheren pH-Wert aufweisen. Dem Fachmann ist bekannt, dass er in einem solchen Fall den pH-Wert des Waschpuffers entsprechend anpassen muss. In den meisten Fällen wird die Bindung zwischen dem Protein und dem Protein A Affinitätsliganden sich jedoch erst unterhalb von pH 4 lösen.

In einem weiteren Aspekt hat der Waschpuffer einen pH-Wert von 6 bis 8.

In einer typischen Ausführungsform kann die Erfindung beispielsweise wie folgt ausgeführt werden:
Protein A-Chromatographie ist in der Regel der erste Reinigungsschritt. Der zellfreie Zellkulturüberstand kann direkt auf die Säule gegeben werden, oder es kann erst eine Konzentrierung über eine Ultrafiltrationsmembran erfolgen, ein so genanntes UF/DF System.

Die Protein A-Säule wird zunächst mit Phosphatpuffer (PBS) Puffer equilibriert, der in den physikochemischen Eigenschaften in etwa dem Beladungspool entspricht. Der Beladungspool besteht aus dem Überstand der Zellkultur, der das zu reinigende Produkt enthält sowie auch zusätzliche Medienbestandteile, die notwendig für das Züchten der angewendeten Säugetierzellen wie zum Beispiel CHO- oder NSO-Zellen sind. Dieser zellfreie Überstand der Zellkultur oder ein Konzentrat davon wird auf die Protein A Säule beladen. Dabei bindet das Zielprotein mit der Protein A-Bindestelle an der Säule. Anschließend wird die Säule mit Equilibrierungspuffer gespült, bis die nicht gebundenen Medienbestandteile und Zellprodukte eluiert sind. Der beschriebene Waschpuffer kann im Anschluss an den Equilibrierungspuffer oder direkt nach der Beladung aufgegeben werden. Die Menge des Waschpuffers ist abhängig vom Maßstab und wird daher relativ zur Größe der Chromatographie-Säule angegeben.

Üblicherweise beträgt das Volumen von Waschpuffern ca. das 2 bis 5fache des Volumens der Chromatographie-Säule (2 - 5 Bett-Volumina, BV). Nach der Applikation des Waschpuffers kann die Säule erneut mit dem Equilibrierungspuffer oder einem anderen Puffer behandelt werden, um keine Bestandteile des Waschpuffers mit dem Produkt zu eluieren. Der hier angewendete Puffer muß einen pH Wert haben, der niedriger als der des Waschpuffers, aber höher als der des Elutionspuffers ist und in Puffersalz und Zusammensetzung dem Elutionspuffer ähnelt. Zur Elution wird ein Puffer mit einem pH-Wert unter pH 4 verwendet, der als Hauptbestandteil zum Beispiel die Salze Acetat oder Citrat enthalten kann. Elutionspuffer können beinhalten ein Acetat in Konzentrationen zwischen 10 mM bis 200 mM, bevorzugt zwischen 20 mM und 100 mM, besonders bevorzugt 50 mM und 100 mM oder Citrat in Konzentrationen zwischen 10 und 200 mM, vorzugsweise zwischen 20 und 100 mM, beide Puffer sollten im oben genannten pH-Bereich unter 4, vorzugsweise zwischen 3 und 4, besonders bevorzugt zwischen 3,4 und 3,6. Zusätzlich können auch Additive wie Arginin oder PVP enthalten sein. Außerdem kann ein Glycinpuffer benutzt werden in Konzentrationen zwischen 10 und 200 mM, vorzugsweise zwischen 25 und 100 mM, mit einem pH-Wert zwischen 2 und 3,5, oder andere Puffer, die geeignet sind, die Bindung zwischen der Fc-Domäne des Antikörpers und dem Protein A zu reduzieren. Das Eluat kann dann im folgenden Prozess weiter aufgearbeitet werden, zum Beispiel Inkubation bei niedrigem pH-Wert oder Neutralisation.

### Beispiele

Es wurden Experimente mit verschiedenen Proteinen durchgeführt, die aus unterschiedlichen Zelllinen stammen (CHO und NS0), die in unterschiedlichen Medien fermentiert wurden, und deren Fc-Teile unterschiedlichen IgG-Subtypen angehören (IgG1, IgG2, IgG4).

Es wurden jeweils Versuchsreihen durchgeführt, bei denen vom Standard vorgehen jeweils nur der oder die Waschpuffer verändert wurden. Es wurde entweder der Equilibrierungspuffer ohne Zusatz aufgegeben, ein Waschpuffer mit einem einzelnen Zusatz, mehrere verschiedene Puffer sequenziell mit jeweils einem Zusatz, oder ein Waschpuffer mit einer Kombination mehrerer Zusätze.

Die aufgetragene Protein-Lösung war dabei für das jeweilige Protein immer gleich. Gemessen wurde in jedem Versuch die Menge an Verunreinigungen im Eluat. Durch Vergleiche zwischen den Versuchen wurden jeweils die Waschpuffer identifiziert, nach denen das Eluat möglichst geringe Mengen der jeweiligen Verunreinigung aufwies. Bei einigen Versuchen wurden auch Ausbeuten, Trübungen und Monomer-Gehälter gemessen.

### Chromatographie

Die Chromatographie-Experimente wurden an einem automatisierten System ÄKTA-FPLC Modell 900 (GE Healthcare) durchgeführt. Für die Versuche wurden vier verschiedene Produkte verwendet. Von den Produkten wurde als Ausgangsmaterial jeweils entweder der zellfreie Kulturüberstand verwendet oder konzentrierter Kulturüberstand, wobei mit einer 50 kD Omega Membran (Pall) zehnfach ankonzentriert und anschließend mit PBS dreifach diafiltriert wurde.

Die eingesetzten Säulen hatten ein Volumen von 1 mL bis 8 mL und enthielten eines der beiden Chromatographie-Gele MabSelect oder MabSelect Xtra (GE Healthcare),

### Puffer

In allen Versuchen wurde als Equilibrierungspuffer ein Phosphatpuffer (PBS) mit 10 mM Phosphat, 5 mM Kaliumchlorid und 140 mM Natriumchlorid bei pH 7,4 verwendet.

Alle Waschpuffer basierten auf einem PBS Puffer (pH 7.4) mit
8 mmol/L Natriummonohydrogenphosphat,
1,5 mmol/L Kaliumhydrogenphosphat,
2,7 mM Kaliumchlorid und
140 mM Natriumchlorid.

Als Zusätze wurden jeweils eingesetzt, einzeln oder in unterschiedlichen Kombinationen (wie in den Abbildungen angegeben):
(1) 860 mmol/L Natriumchlorid (Gesamtgehalt 1 mol/L Natriumchlorid)
(2) 0,25 % (w/V) Polyvinylpyrrolidon (PVP)
(3) 15 % (V/V) Isopropanol
(4) 0,5 mol/L L-Arginin

Zur Elution wird je nach Produkt ein unterschiedlicher Puffer verwendet, mit unterschiedlichen Acetatkonzentrationen und pH. Folgende Konzentrationen wurden benutzt:
BI-Mab 06a: 100 mM Acetat ph 3,4
BI-Mab 1003a: 50 mM Acetat ph 3,4
BI-Mab 1001 b: 50 mM Acetat ph 3,4
BI-Mab 07c: 50 mM Acetat ph 3,6

### Analysen

Die Eluate der Versuche wurden hinsichtlich ihres Gehalts an Verunreinigungen verglichen und die Ausbeuten bestimmt.

Zur Bestimmung der Menge an Zellbestandteilen wurde ein generischer Sandwich-ELISA verwendet, der Wirtszell-Proteine als Summenparameter bestimmt. Für den Assay werden polyklonale Detektions-Antikörper verwendet.

Zur Bestimmung des Monomeranteils wurde das System Agilent Series HPLC 1200 (waters) und je nach Protein eine TSK 3000SW oder eine TSK 3000SWXL Säule (TosoH) verwendet. Die isokratische Methode läuft bei einem Fluss von 1 mL/min mit einem Tris-Puffer bei pH 7,0.

Die Bestimmung der DNA erfolgt nach der Threshold-Methode (Kung, V.T. et al., Picogram Quantitation of Total DNA Using DNA-Binding Proteins in a Silicon Sensor-Based System, Anal. Biochem. 1990, 187, 220-227).

Zur Durchführung einer SDS-PAGE wird ein Phast-System (GE Healthcare) verwendet. Aufgetrennt werden die Proben mit einem Phast SDS-Gel (4%-15%, GE). Die Färbung erfolgt nach der Heukeshoven Silberfärbung (Heukeshoven, Dernick 1988, Electrophoresis 9 (1), Seiten 28-32).

Zur Bestimmung der Menge an Antikörper in der Beladung und im Eluat wurde eine PA 2-1001-00 ProteinA Säule (Applied Biosystems) und ein Agilent Series 1200 HPLC-System (waters) verwendet. Die Bindung und Elution erfolgt über einen Gradienten von pH 7,4 bis pH 2,8 im PBS Puffersystem, die Auswertung anhand einer externen Kalibriergerade des jeweiligen Antikörpers.

Die Messung der Trübung erfolgt im 2100AN Turbidimeter (Hach) nach Kalibration mit Trübungsstandards des Herstellers.

### Ergebnisse

### Experimente mit BI-MAb 06a, ein Antikörper der Subklasse IgG1

Die Versuche zeigen einen deutlichen Einfluss der Waschung auf Ausbeute, Monomer, Trübung und Wirtszell-Proteine (Host Cell Proteins, HCP).

In den Qualitätskriterien Ausbeute, Monomer-Gehalt und HCP-Abreicherung zeigt das Eluat jeweils nach der Waschung mit dem Puffer den besten Wert, der alle vier Zusätze enthält. Außerdem führt die Kombination der Zusätze zu einer erheblichen Reduktion von Trübungen, und zwar sowohl bei der Kombination der drei Bestandteile 0,86 mol/L Natriumchlorid, 0,25% (w/V) PVP und 15% (V/V) Isopropanol, als auch bei der Kombination aller vier Zusätze 0,86 mol/L Natriumchlorid, 0,25% (w/V) PVP, 15% (V/V) Isopropanol und 0,5 mol/L Arginin.

### Experimente mit BI-MAb 1003a, ein Antikörper der Subklasse IgG1

Die beste HCP Abreicherung wird mit dem Waschpuffer erzielt, der alle vier zusätzlichen Komponenten enthält. Ebenfalls gute Werte liefert der Waschpuffer mit der Kombination aus Natriumchlorid, PVP und Isopropanol. Bei der Waschung mit Puffern, die die vier Komponenten einzeln enthalten, wird mehr HCP im Eluat erhalten.

Auch bei den Trübungen führt die Kombination der Wasch-Substanzen in einem Puffer zu einem besseren Wert.

### Experimente mit BI-MAb 07c, ein Antikörper der Subklasse IgG4

Die Eluate der Versuche der einzelnen Waschpuffer haben höhere Werte an HCP als die Eluate der Versuche mit kombinierten Waschpuffern.

Experimente mit BI-MAb 1001 b, ein Antikörper der Subklasse IgG2 Die Eluate der Versuche der einzelnen Waschpuffer haben höhere Werte an HCP als die Eluate der Versuche mit kombinierten Waschpuffern.

Die vier Experimente zeigen, dass die Kombination aus den vier Zusätzen Natriumchlorid, PVP, Isopropanol und Arginin in einem einzigen Waschpuffer vorteilhaft gegenüber der Anwendung von Waschpuffern mit einzelnen Zusätzen in Bezug auf den Gehalt an Host Cell Proteinen (HCP) im Eluat ist.

Die Anwendung des beschriebenen Waschpuffers zeigt ebenfalls, dass Trübungen im Eluat deutlich reduziert sind. Dabei verbessern sich auch der Anteil an Monomer und die Ausbeute (BI-MAb 06a), oder sie bleiben zumindest gleich (BI-MAb 1003a).

Der beschriebene Waschpuffer ist daher geeignet, das Produktionsverfahren für Proteine erheblich zu verbessern. Durch die Einführung der Kombination der Waschpuffer werden Verunreinigungen abgereichert, die andernfalls mit einem zusätzlichen Reinigungsschritt entfernt werden müssten.

## Patentansprüche

1. Verfahren zur Abreicherung von Verunreinigungen aus einer Zusammensetzung, die ein Protein enthält, das die Fc-Domäne eines Immunglobulins aufweist (Zielprotein), durch Protein-A-Chromatographie, mit den Schritten:
a. Anwendung einer mobilen Phase, die das Zielprotein enthält, auf eine stationäre Phase, die Protein A enthält, unter Bedingungen, unter denen das Zielprotein an die stationäre Phase bindet;
b. Anwendung eines Waschpuffers mit einem pH-Wert von 5 bis 8 als mobiler Phase, enthaltend
i. Arginin in einer Konzentration von 0,1 - 1 mol/l,
ii. Natriumchlorid in einer Konzentration von 0,2 bis 2 mol/l,
iii. einen Alkohol, ausgewählt aus der Gruppe bestehend aus Isopropanol, n-Propanol, und Ethanol, in einer Konzentration von 5 - 30 % v/v, und
iv. Polyvinylpyrrolidon oder ein Detergenz in einer Konzentration von 0.05 - 2 % (w/v);
c. Anwendung eines Elutionspuffers als mobiler Phase unter Bedingungen, unter denen das Zielprotein von der stationären Phase eluiert wird.

2. Verfahren nach Anspruch 1, **dadurch gekennzeichnet, dass** die Argininkonzentration im Waschpuffer 0,4 - 0,6 mol/l beträgt.

3. Verfahren nach einem der vorangehenden Ansprüche, **dadurch gekennzeichnet, dass** die Natriumchloridkonzentration im Waschpuffer 0,9 - 1,1 mol/l beträgt.

4. Verfahren nach einem der vorangehenden Ansprüche, **dadurch gekennzeichnet, dass** der Alkohol im Waschpuffer Isopropanol in einer Konzentration von 10 - 20 % (w/v) ist.

5. Verfahren nach einem der vorangehenden Ansprüche, **dadurch gekennzeichnet, dass** Polyvinylpyrrolidon (PVP) in einer Konzentration von 0,1 - 2 % (w/v) ist.

6. Verfahren nach einem der vorangehenden Ansprüche, **dadurch gekennzeichnet, dass** das Detergens ein Polyoxyethylen-sorbitan-monolaurat in einer Konzentration von 0,05 - 2 % (w/v) ist.

7. Verfahren nach einem der vorangehenden Ansprüche, **dadurch gekennzeichnet, dass** es sich bei den Verunreinigungen um Wirtszellproteine (HCP) handelt.

8. Waschpuffer für die Affinitätschromatographie mit einem pH-Wert von 5 bis 8, enthaltend
i. Arginin in einer Konzentration von 0,1 - 1 mol/l,
ii. Natriumchlorid in einer Konzentration von 0,2 bis 2 mol/l,
iii. einen Alkohol, ausgewählt aus der Gruppe bestehend aus Isopropanol, n-Propanol, und Ethanol, in einer Konzentration von 5 - 30 % (w/v), und
iv. Polyvinylpyrrolidon oder ein Detergens in einer Konzentration von 0.05 - 2 % (w/v);

## Claims

1. Method of depleting impurities from a composition that contains a protein which comprises the Fc domain of an immunoglobulin (target protein), by protein-A chromatography, comprising the steps of:
a. applying a mobile phase which contains the target protein to a stationary phase which contains protein A, under conditions in which the target protein binds to the stationary phase;
b. applying a washing buffer with a pH of between 5 and 8 as mobile phase, containing
i. arginine in a concentration of 0.1 - 1 mol/l,
ii. sodium chloride in a concentration of 0.2 to 2 mol/l,
iii. an alcohol selected from among isopropanol, n-propanol and ethanol, in a concentration of 5 - 30 % (v/v) and
iv. polyvinylpyrrolidone or a detergent in a concentration of 0.05 - 2 % (w/v);
c. using an elution buffer as mobile phase under conditions in which the target protein is eluted from the stationary phase.

2. Method according to claim 1, **characterised in that** the arginine concentration in the washing buffer is 0.4 - 0.6 mol/l.

3. Method according to one of the preceding claims, **characterised in that** the sodium chloride concentration in the washing buffer is 0.9 - 1.1 mol/l.

4. Method according to one of the preceding claims, **characterised in that** the alcohol in the washing buffer is isopropanol in a concentration of 10 - 20 % (w/v).

5. Method according to one of the preceding claims, **characterised in that** polyvinylpyrrolidone (PVP) is in a concentration of 0.1 - 2 % (w/v).

6. Method according to one of the preceding claims, **characterised in that** the detergent is a polyoxyethylene-sorbitan-monolaurate in a concentration of 0.05 - 2 % (w/v).

7. Method according to one of the preceding claims, **characterised in that** the impurities are host cell proteins (HCP).

8. Washing buffer for the affinity chromatography with a pH of 5 to 8, containing
i. arginine in a concentration of 0.1 - 1 mol/l,
ii. sodium chloride in a concentration of 0.2 to 2 mol/l,
iii. an alcohol selected from among isopropanol, n-propanol and ethanol, in a concentration of 5 - 30 % (w/v), and
iv. polyvinylpyrrolidone or a detergent in a concentration of 0.05 - 2 % (w/v).

## Revendications

1. Procédé de réduction d'impuretés dans une composition qui contient une protéine qui présente le domaine Fc d'une immunoglobuline (protéine cible), par chromatographie de la protéine A, comportant les étapes de :
a. l'utilisation d'une phase mobile qui contient la protéine cible, sur une phase stationnaire qui contient la protéine A, dans des conditions dans lesquelles la protéine cible se lie à la phase stationnaire ;
b. l'utilisation d'un tampon de lavage ayant un pH de 5 à 8 comme phase mobile, contenant
i. de l'arginine en une concentration de 0,1 à 1 mole/l,
ii. du chlorure de sodium en une concentration de 0,2 à 2 moles/l,
iii. un alcool choisi dans le groupe comprenant l'isopropanol, le n-propanol et l'éthanol, en une concentration de 5 à 30 % v/v, et
iv. de la polyvinylpyrrolidone ou un détergent en une concentration de 0,05 à 2 % (p/v) ;
c. l'utilisation d'un tampon d'élution comme phase mobile dans des conditions dans lesquelles la protéine cible est éluée de la phase stationnaire.

2. Procédé selon la revendication 1, **caractérisé en ce que** la concentration d'arginine dans le tampon de lavage est de 0,4 à 0,6 mole/l.

3. Procédé selon l'une des revendications précédentes, **caractérisé en ce que** la concentration de chlorure de sodium dans le tampon de lavage est de 0,9 à 1,1 mole/l.

4. Procédé selon l'une des revendications précédentes, **caractérisé en ce que** l'alcool dans le tampon de lavage est l'isopropanol en une concentration de 10 à 20 % (p/v).

5. Procédé selon l'une des revendications précédentes, **caractérisé en ce que** la polyvinylpyrrolidone (PVP) est en une concentration de 0,1 à 2 % (p/v).

6. Procédé selon l'une des revendications précédentes, **caractérisé en ce que** le détergent est un monolaurate de polyoxyéthylène sorbitane en une concentration de 0,05 à 2 % (p/v).

7. Procédé selon l'une des revendications précédentes, **caractérisé en ce que** les impuretés sont des protéines des cellules hôtes (HCP).

8. Tampon de lavage pour la chromatographie d'affinité présentant un pH de 5 à 8, contenant
i. de l'arginine en une concentration de 0,1 à 1 mole/l,
ii. du chlorure de sodium en une concentration de 0,2 à 2 moles/l,
iii. un alcool choisi dans le groupe comprenant l'isopropanol, le n-propanol et l'éthanol, en une concentration de 5 à 30 % (p/v), et
iv. de la polyvinylpyrrolidone ou un détergent en une concentration de 0,05 à 2 % (p/v).
